(19) Europäisches Patentamt
European Patent Office
Office européen des brevets

(11) Veröffentlichungsnummer: **0 028 759**
**B1**

(12) # EUROPÄISCHE PATENTSCHRIFT

(45) Veröffentlichungstag der Patentschrift: **04.08.82**

(51) Int. Cl.³: **C 07 C 69/716,** C 07 C 67/317

(21) Anmeldenummer: **80106591.3**

(22) Anmeldetag: **25.10.80**

(54) **Verfahren zur Herstellung von Pivaloylessigsäureestern.**

(30) Priorität: **12.11.79 DE 2945604**

(43) Veröffentlichungstag der Anmeldung: **20.05.81 Patentblatt 81/20**

(45) Bekanntmachung des Hinweises auf die Patenterteilung: **04.08.82 Patentblatt 82/31**

(84) Benannte Vertragsstaaten: **BE CH DE FR GB IT LI**

(56) Entgegenhaltungen: **US A 2 527 306**

(73) Patentinhaber: **BAYER AG, Zentralbereich Patente, Marken und Lizenzen, D-5090 Leverkusen 1, Bayerwerk (DE)**

(72) Erfinder: **Werner, Friedrich, Dr., Petersenstrasse 1, D-5000 Köln 91 (DE)**
Erfinder: **Blank, Heinz Ulrich, Dr., Am Geusfelde 35, D-5068 Odenthal (DE)**

EP 0 028 759 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Pivaloylessigsäureestern

Die Erfindung betrifft ein Verfahren zur Herstellung von Pivaloylessigsäureestern durch Decarbonylierung von Pivaloylbrenztraubensäureestern.

Es ist bekannt, Pivaloylessigsäuremethylester durch 5½stündiges Erhitzen einer Mischung aus Pivaloylbrenztraubensäuremethylester mit etwa 11 Gew.-%, bezogen auf den Pivaloylbrenztraubensäureester, an gepulvertem Glas auf etwa 175°C in einer Ausbeute von 80% der theoretischen Ausbeute zu erhalten (US-Patent 2 527 306). Dieses Verfahren birgt die Gefahr in sich, daß beim plötzlichen Einsetzen der Reaktion kurzfristig große Mengen Kohlenmonoxid gebildet werden. Der dabei eintretende plötzliche Druckanstieg kann zur Zerstörung der Apparatur führen. Darüber hinaus ist das freiwerdende Kohlenmonoxid wegen seiner Giftigkeit gefährlich.

Es wurde nun ein Verfahren zur Herstellung von Pivaloylessigsäureestern, deren Alkoholkomponente einen $C_1-C_{10}$-Alkylrest oder einen $C_3-C_8$-Cycloalkylrest enthält, durch Decarbonylierung von Pivaloylbrenztraubensäureestern, deren Alkoholkomponente die genannte Bedeutung hat, bei erhöhter Temperatur gefunden, das dadurch gekennzeichnet ist, daß man zunächst höchstens 30% des umzusetzenden Pivaloylbrenztraubensäureesters in Gegenwart von 0,01 bis 5 Gew.-%, bezogen auf die Gesamtmenge des umzusetzenden Pivaloylbrenztraubensäureesters, eines Metalles der ersten bis achten Nebengruppe des Periodensystems der Elemente (Mendelejew) in metallischer und/oder oxidischer Form zur Reaktion bringt und nach Einsetzen der Decarbonylierungsreaktion die verbleibende Menge des Pivaloylbrenztraubensäureesters entsprechend dem Umsatz zugibt.

Als Pivaloylbrenztraubensäureester werden im erfindungsgemäßen Verfahren solche der Formel

$$(CH_3)_3C—CO—CH_2—CO—COOR \qquad (I)$$

eingesetzt, in der

R für $C_1$–$C_{10}$-Alkyl oder $C_3$–$C_8$-Cycloalkyl steht.

Als Alkyl sei ein geradkettiger oder verzweigter aliphatischer Kohlenwasserstoffrest mit 1 bis 10, bevorzugt 1 bis 4, besonders bevorzugt 1 bis 2 Kohlenstoffatomen genannt, wie Methyl, Ethyl, Propyl, Isopropyl, Butyl, Isobutyl, Hexyl, Octyl oder Decyl.

Als Cycloalkyl sei beispielsweise ein gesättigter carbocyclischer, gegebenenfalls durch Methyl- oder Ethylgruppen substituierter Kohlenwasserstoffrest mit 3 bis 8, bevorzugt 5 bis 6 Kohlenstoffatomen genannt, beispielsweise Cyclopropyl, Cyclobutyl, Cyclopentyl, Methylcyclopentyl, Ethyl-cyclopentyl, Cyclohexyl, Methyl-cyclohexyl, Ethyl-cyclohexyl, Cycloheptyl oder Cyclooctyl.

Das erfindungsgemäße Verfahren wird bei erhöhter Temperatur durchgeführt. Als solche sei beispielsweise eine Temperatur von 130 bis 230° C, bevorzugt 170 bis 200° C, genannt.

Im erfindungsgemäßen Verfahren wird die Decarbonylierung des Pivaloylbrenztraubensäureesters in Gegenwart von 0,01 bis 5 Gew.-%, bevorzugt 0,2 bis 1 Gew.-% eines Metalles der ersten bis achten Nebengruppe des Periodensystems der Elemente (Mendelejew) in metallischer und/oder oxidischer Form durchgeführt. Als solche Metalle seien beispielsweise genannt: Kupfer, Silber, Zink, Cadmium, Scandium, Yttrium, Titan, Zirkon, Vanadium, Niob, Chrom, Molybdän, Wolfram, Mangan, Eisen, Kobalt, Nickel, Ruthenium, Rhodium und Palladium.

Die Metalle können in ihrer metallischen und/oder oxidischen Form als solcher oder auf geeigneten Trägern eingesetzt werden. Ebenso können Mischungen der Metalle in ihrer metallischen und/oder oxidischen Form in beliebiger Zusammensetzung verwendet werden. Ebenso können Salze oder andere Verbindungen der genannten Metalle eingesetzt werden, wenn sich diese Salze oder anderen Verbindungen unter den Bedingungen des erfindungsgemäßen Verfahrens in die metallische oder die oxidische Form umwandeln.

In bevorzugter Weise wird im erfindungsgemäßen Verfahren in Gegenwart eines Metalles aus der Gruppe der Eisenmetalle Eisen, Kobalt und Nickel in seiner metallischen und/oder oxidischen Form gearbeitet.

Ganz besonders bevorzugte für das erfindungsgemäße Verfahren sind Eisen und eisenhaltige Metalle, sowie deren Oxide oder Gemische von Eisen oder eisenhaltigen Metallen mit ihren Oxiden. Beispielsweise sei hierfür gemahlenes, gegebenenfalls durch andere Metalle verunreinigtes, gegebenenfalls teilweise oxidiertes Eisen, wie Eisenschrott, genannt.

Im erfindungsgemäßen Verfahren werden zunächst höchstens 30% des umzusetzenden Pivaloylbrenztraubensäureesters in Gegenwart eines der genannten Metalle in metallischer und/oder oxidischer Form auf die gewünschte Reaktionstemperatur gebracht. Als höchstens 30% der Menge des umzusetzenden Pivaloylbrenztraubensäureesters sei beispielsweise eine solche von 1 bis 30%, bevorzugt 2 bis 20%, genannt. Nach dem Einsetzen der Reaktion, das durch die Entwicklung von Kohlenmonoxid gekennzeichnet ist, wird die verbleibende Menge des Pivaloylbrenztraubensäureesters entsprechend dem Umsatz in der fortschreitenden Reaktion portionsweise oder kontinuierlich zugegeben. Diese Zugabe erfolgt beispielsweise in einer Menge von 0,2 bis 4 Mol, bevorzugt 0,5 bis 1,5

Mol, besonders bevorzugt 0,8 bis 1,2 Mol, Pivaloylbrenztraubensäureester pro Mol decarbonylierten Pivaloylbrenztraubensäureester.

Mit Hilfe des erfindungsgemäßen Verfahrens können Pivaloylessigsäureester der Formel

$$(CH_3)_3C-CO-CH_2-COOR \qquad (II)$$

in der

R für Alkyl oder Cycloalkyl steht,

hergestellt werden. Hierbei haben Alkyl und Cycloalkyl den gleichen Bedeutungsumfang wie in Formel (I).

Die Reaktion des erfindungsgemäßen Verfahrens sei anhand der Herstellung des Pivaloylessigsäuremethylesters durch Decarbonylierung des Pivaloylbrenztraubensäuremethylesters durch die folgende Formelgleichung dargestellt:

$$(CH_3)_3C-CO-CH_2-CO-COOCH_3 \rightarrow (CH_3)_3C-CO-CH_2-COOCH_3+CO$$

Das erfindungsgemäße Verfahren wird im allgemeinen ohne Verwendung eines Lösungsmittels durchgeführt. Es ist jedoch auch möglich, ein unter den Reaktionsbedingungen stabiles Lösungsmittel zu verwenden.

Die nach dem erfindungsgemäßen Verfahren hergestellten Pivaloylessigsäureester können weiter mit äquimolaren Mengen Anilin oder substituierten Anilinen durch einstündiges Erhitzen in siedendem Xylol zu den gegebenenfalls substituierten $\alpha$-Pivaloylacetaniliden umgesetzt werden, die Kuppler für gelbe Farben in der Farbphotographie sind (US-Patent 3 265 506).

Es ist überraschend, daß in Gegenwart der genannten Metalle in metallischer und/oder oxidischer Form ohne weitergehende Zersetzung der an der Reaktion beteiligten Stoffe hohe Ausbeuten an Pivaloylessigsäureestern erzielt werden, die über den Ausbeuten des Standes der Technik liegen.

Das erfindungsgemäße Verfahren benötigt durch den Einsatz der genannten Metalle in metallischer und/oder oxidischer Form als Hilfsstoffe weniger Hilfsstoffe als Verfahren nach dem Stande der Technik. Weiterhin wird im erfindungsgemäßen Verfahren stets nur eine kleine, kontrollierbare Menge an Pivaloylbrenztraubensäureester decarbonyliert. Trotzdem werden hohe Raum-Zeit-Ausbeuten erreicht, die bei der bevorzugten Form des erfindungsgemäßen Verfahrens besser sind als die des Standes der Technik.

## Beispiel 1

40 g Pivaloylbrenztraubensäureethylester werden mit 4 g Eisenspänen auf 190°C erhitzt. Nach 0,5 Stunden sind 3 l CO entstanden. Innerhalb von 4 1/2 Stunden tropft man weitere 360 g Ester ein, wobei weitere 46 l Gas entstehen. Man läßt abkühlen und destilliert dann aus dem Reaktionsgemisch bei $Kp_{18}$: 78°C 295 g (85,8% der theoretischen Ausbeute) Pivaloylessigsäureethylester mit einer Reinheit von 99% (nach gaschromatographischer Analyse). $n_D^{25}$: 1,4310.

## Beispiel 2

20 g Pivaloylbrenztraubensäuremethylester werden unter Rühren mit 2 g Eisenspänen auf 180°C erhitzt. Nach ca. 40 Minuten sind 1,5 l CO entstanden. Innerhalb von 3 1/2 Stunden werden 180 g Ester hinzugetropft. Insgesamt werden 25,2 l CO gebildet. Destillation bei $Kp_{20}$: 82°C liefert 157,3 g Pivaloylessigsäuremethylester (92,1% der theoretischen Ausbeute) mit einer Reinheit von 99,2% (nach gaschromatographischer Analyse). $_D^{25}$ = 1,4315.

## Beispiele 3–9

Bei analoger Arbeitsweise wie in Beispiel 2 werden bei Anwendung der in der Tabelle genannten Katalysatoren bzw. Temperaturen folgende Ergebnisse erzielt:

| Beispiel | Katalysator | Temp. | Vorgelegte Menge Ester | Zugetropfte Menge Ester | Gesamtreaktionszeit | Ausbeute | Selektivität |
|---|---|---|---|---|---|---|---|
| | | °C | g | g | h | % d. Th. | |
| 3 | Fe-Späne | 190 | 40 | 160 | 3½ | 90,4 | 90,4 |
| 4 | Cu-Pulver | 195 | 40 | 160 | 13½ | 75,5 | 75,5 |
| 5 | Ni auf $SiO_2$ | 182 | 40 | 160 | 5½ | 86,1 | 86,1 |
| 6 | Mn-Pulver | 190 | 40 | 160 | 8 | 79,5 | 79,5 |
| 7 | Cr-Pulver | 200 | 40 | 160 | 8 | 61,5 | 87,7 |
| 8 | Mo-Pulver | 190 | 40 | 160 | 5½ | 86,9 | 86,9 |
| 9 | Co-Pulver | 190 | 40 | 160 | 8 | 84,0 | 84,0 |

**Patentansprüche**

1. Verfahren zur Herstellung von Pivaloylessigsäureestern, deren Alkoholkomponente einen $C_1-C_{10}$-Alkylrest oder einen $C_3-C_8$-Cycloalkylrest enthält, durch Decarbonylierung von Pivaloylbrenztraubensäureestern, deren Alkoholkomponente die genannte Bedeutung hat, bei erhöhter Temperatur, dadurch gekennzeichnet, daß man zunächst höchstens 30% des umzusetzenden Pivaloylbrenztraubensäureesters in Gegenwart von 0,01 bis 5 Gew.-%, bezogen auf die Gesamtmenge des umzusetzenden Pivaloylbrenztraubensäureesters, eines Metalles der ersten bis achten Nebengruppe des Periodensystems der Elemente (Mendelejew) in metallischer und/oder oxidischer Form aufheizt und nach Einsetzen der Reaktion die verbleibende Menge des Pivaloylbrenztraubensäureesters entsprechend dem Umsatz zugibt.

2. Verfahren nach Anspruch 1, dadurch gekennzeichnet, daß man zunächst 2 bis 20% des umzusetzenden Pivaloylbrenztraubensäureesters vorlegt und den Rest entsprechend dem Umsatz zugibt.

3. Verfahren nach Anspruch 1 und 2, dadurch gekennzeichnet, daß man in Gegenwart eines Metalles in metallischer und/oder oxidischer Form aus der Gruppe der Eisenmetalle Eisen, Kobalt und Nickel arbeitet.

4. Verfahren nach Anspruch 1 bis 3, dadurch gekennzeichnet, daß man in Gegenwart von Eisen in metallischer und/oder oxidischer Form arbeitet.

**Claims**

1. Process for the preparation of pivaloylacetic acid esters, the alcohol component of which contains a $C_1-C_{10}$-alkyl radical or a $C_3-C_8$-cycloalkyl radical, by decarbonylation of pivaloylpyruvic acid esters, the alcohol component of which has the indicated meaning, at elevated temperature, characterised in that at most 30% of the pivaloylpyruvic acid ester to be converted is first heated in the presence of 0.01 to 5% by weight, relative to the total amount of pivaloylpyruvic acid ester to be converted, of a metal of sub-groups one to eight of the periodic system of the elements (Mendeleev) in metallic and/or oxidic form, and after the reaction has started, the remaining amount of the pivaloylpyruvic acid ester is added according to the rate at which it is converted.

2. Process according to Claim 1, characterised in that 2 to 20% of the pivaloylpyruvic acid ester to be converted is first introduced and the remainder is added, according to the rate at which it is converted.

3. Process according to Claims 1 and 2, characterised in that it is carried out in the presence of a metal, in metallic and/or oxidic form, of the iron group, that is to say iron, cobalt or nickel.

4. Process according to Claims 1 to 3, characterised in that it is carried out in the presence of iron in metallic and/or oxidic form.

**Revendications**

1. Procédé de fabrication d'esters d'acide pivaloylacétique dont le composant alcoolique contient un radical alcoyle en $C_1-C_{10}$ ou un radical cycloalcoyle en $C_3-C_8$, par décarbonylation d'esters d'acide pivaloylpyruvique dont le composant alcoolique a la signification indiquée, à température élevée, caractérisé en ce que tout d'abord on chauffe au maximum 30% de l'ester d'acide pivaloylpyruvique à faire réagir en présence de 0,01 à 5% en poids, par rapport à la quantité totale de l'ester d'acide pivaloylpyruvique à faire réagir, d'un métal du premier au huitième sous-groupe du système périodique des éléments (Mendelejew) sous une forme métallique et/ou oxydée et en ce qu'après mise en train de la réaction on ajoute la quantité restante de l'ester d'acide pivaloylpyruvique en rapport avec la conversion.

2. Procédé selon la revendication 1, caractérisé en ce qu'on ajoute d'avance tout d'abord 2 à 20% de l'ester d'acide pivaloylpyruvique à faire réagir et ajoute le restant en rapport avec la conversion.

3. Procédé selon les revendications 1 et 2, caractérisé en ce qu'on opère en présence d'un métal sous la forme métallique et/ou oxydée appartenant au groupe des métaux ferreux fer, cobalt et nickel.

4. Procédé selon les revendications 1 à 3, caractérisé en ce qu'on opère en présence de fer sous la forme métallique et/ou oxydée.